# EUROPEAN PATENT SPECIFICATION

(11) **EP 2 180 062 B1**
(45) Date of publication and mention of the grant of the patent: **03.10.2012**
(21) Application number: 08827253.9
(22) Date of filing: 11.08.2008
(51) Int. Cl.: C12Q 1/68, C12N 15/09

(54) **METHOD OF DETECTING TARGET SUBSTANCE**
VERFAHREN ZUR ERKENNUNG EINER ZIELSUBSTANZ
PROCÉDÉ DE DÉTECTION D'UNE SUBSTANCE CIBLE

(30) Priority: 14.08.2007 JP 2007211524
(43) Date of publication of application: 28.04.2010
(73) Proprietor: Eisai R&D Management Co., Ltd., Tokyo 112-8088 (JP)
(72) Inventor: USUI, Mitsugu, Kawasaki-shi Kanagawa 210-0855 (JP); HAKII, Chikako, Kawasaki-shi Kanagawa 210-0855 (JP)
(74) Representative: Grünecker, Kinkeldey, Stockmair & Schwanhäusser
(86) International application number: PCT/JP2008/064423
(87) International publication number: WO 2009/022683

(56) References cited:
- EP-A1- 1 595 953
- WO-A1-2006/028162
- WO-A1-2006/093097

### SEQUENCE LISTING

<110> Eisai R&D Management Co., Ltd.
<120> Method for detecting target substance
<130> 77422-P-PCT
<150> JP 2007-211524
   <151> 2007-08-14
<160> 14
<170> PatentIn version 3.1
<210> 1
   <211> 42
   <212> DNA
   <213> Artificial
<220>
   <223> Description of Artificial Sequence; Synthetic
<220>
   <221> misc_feature
   <222> (1)..(1)
   <223> digoxigenin attached at the 5'end
<400> 1
   catctctgct ggtccctcgg ctgcgtcggt tcgccataga cg 42
<210> 2
   <211> 42
   <212> DNA
   <213> Artificial
<220>
   <223> Description of Artificial Sequence; Synthetic
<220>
   <221> misc_feature
   <222> (1)..(1)
   <223> digoxigenin attached at the 5'end
<400> 2
   gcacattcac accgcgacgc agccgaggcc tgacctctat gc 42
<210> 3
   <211> 28
   <212> DNA
   <213> Artificial
<220>
   <223> Description of Artificial Sequence; Synthetic
<400> 3
   gaccagcaga gatgcgtcta tggcgaac 28
<210> 4
   <211> 28
   <212> DNA
   <213> Artificial
<220>
   <223> Description of Artificial Sequence; Synthetic
<400> 4
   cggtgtgaat gtgcgcatag aggtcagg 28
<210> 5
   <211> 105
   <212> DNA
   <213> Artificial
<220>
   <223> Description of Artificial Sequence; Synthetic
<400> 5
   ttcgggaaac cggagctaat accggataat attttgaacc gcatggttca aaagtgaaag 60
   acggtcttgc tgtcacttat agatggatcc gcgctgcatt agcta 105
<210> 6
   <211> 49
   <212> DNA
   <213> Artificial
<220>
   <223> Description of Artificial Sequence; Synthetic
<400> 6
   gcatagaggt cagggaccag cagagatgat ctataagtga cagcaagac 49
<210> 7
   <211> 42
   <212> DNA
   <213> Artificial
<220>
   <223> Description of Artificial Sequence; Synthetic
<220>
   <221> misc_feature
   <222> (42)..(42)
   <223> Aminolink attached at the 3'end
<400> 7
   cgtctttcac ttttgaacca tgcggttcaa aatattatcc gg 42
<210> 8
   <211> 77
   <212> DNA
   <213> Artificial
<220>
   <223> Description of Artificial Sequence; Synthetic
<400> 8
   gcatagaggt cagggaccag cagagatggc atagaggtca gggaccagca gagatgatct 60
   ataagtgaca gcaagac 77
<210> 9
   <211> 63
   <212> DNA
   <213> Artificial
<220>
   <223> Description of Artificial Sequence; Synthetic
<400> 9
   gaccagcaga gatgcgtcta tggcgaaccg gtgtgaatgt gcatctataa gtgacagcaa 60
   gac 63
<210> 10
   <211> 35
   <212> DNA
   <213> Artificial
<220>
   <223> Description of Artificial Sequence; Synthetic
<400> 10
   gaccagcaga gatgatctat aagtgacagc aagac 35
<210> 11
   <211> 63
   <212> DNA
   <213> Artificial
<220>
   <223> Description of Artificial Sequence; Synthetic
<400> 11
   catctctgct ggtccctcgg ctgcgtcggt tcgccataga cgatctataa gtgacagcaa 60
   gac 63
<210> 12
   <211> 63
   <212> DNA
   <213> Artificial
<220>
   <223> Description of Artificial Sequence; Synthetic
<400> 12
   gaccagcaga gatgggtgcc gtcgctgccg tctatggcga acatctataa gtgacagcaa 60
   gac 63
<210> 13
   <211> 42
   <212> DNA
   <213> Artificial
<220>
   <223> Description of Artificial Sequence; Synthetic
<220>
   <221> misc_feature
   <222> (1)..(1)
   <223> digoxigenin attached at the 5'end
<400> 13
   catctctgct ggtccctcgg ctgcgtcgag tgacagcaag ac 42
<210> 14
   <211> 28
   <212> DNA
   <213> Artificial
<220>
   <223> Description of Artificial Sequence; Synthetic
<220>
   <221> misc_feature
   <222> (1)..(1)
   <223> digoxigenin attached at the 5'end
<400> 14
   gaccagcaga gatggtcttg ctgtcact 28

## Description

### Technical Field

The present invention relates to a kit for detection of a target substance including a dimer probe and a crosslinking probe which form a probe polymer, and a novel assist probe, a method of detecting a target substance using the kit for detection, and a method of forming a signal probe polymer.

### Background Art

For a method of amplifying a signal without using an enzyme, Patent Document 1 discloses a method of amplifying a signal (hereinafter referred to as a PALSAR method) in which dimer probes each having a structure of the following formula (1) and two kinds of crosslinking probes having structures of the following formulae (2) and (3) or two kinds of crosslinking probes having structures of the following formulae (4) and (5) are used and an assembly substance (polymer) is formed by a self-assembly reaction of the dimer probes and the crosslinking probes.

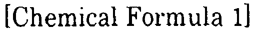

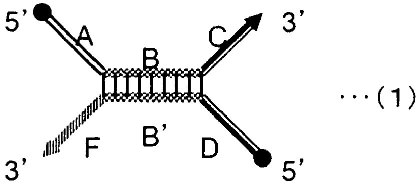

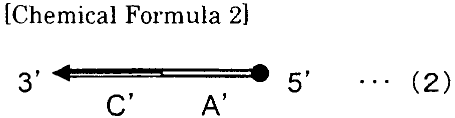

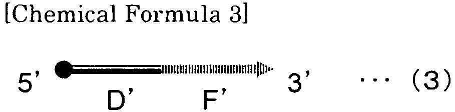

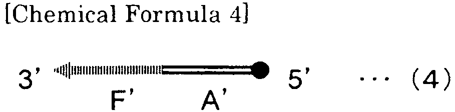

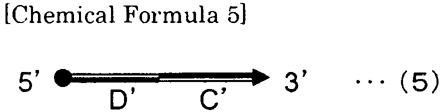

In the above formulae (1) to (5), a region A and a region A', a region B and a region B', a region C and a region C', a region D and a region D', and a region F and a region F' have complementary base sequences, respectively, and a self-assembly substance having a structure represented by the following formula (6) is formed by binding the dimer probes and the crosslinking probes. The signal probe polymer as used herein refers to a nucleic acid probe polymer including the self-assembly substance formed of the dimer probes and the crosslinking probes.

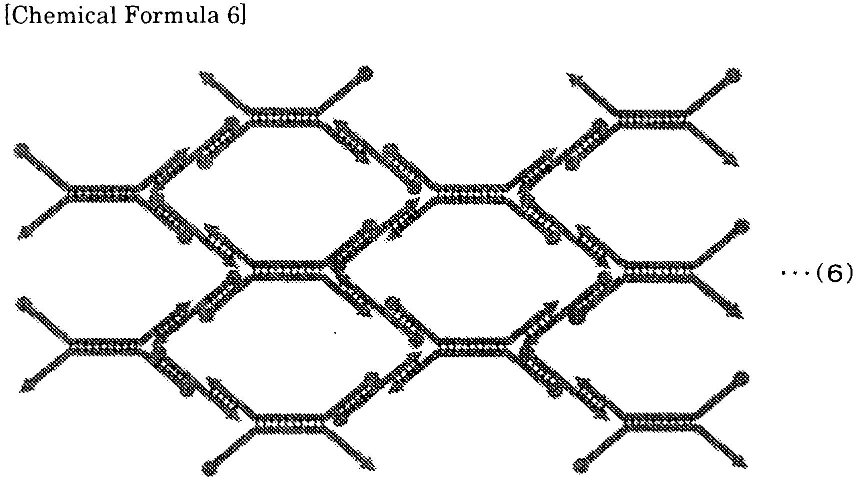

Patent Document 1 discloses a method of detecting a gene by a ligation reaction using a preliminarily cleaved dimer-forming probe as a method of detecting a gene utilizing the PALSAR method, but the method requires multiple cycles of ligation reactions.

Patent Documents 2 and 3 each disclose a method of detecting a target gene with high sensitivity by forming a complex of the target gene and a probe polymer utilizing the PALSAR method and detecting the probe polymer. For a method of forming a signal probe polymer on a target gene, Patent Document 2 discloses a method of designing one region at the end of a dimer-forming probe so as to have a base sequence complementary to the target gene. Further, Patent Document 3 discloses a method of using a probe (assist probe) separately having both a base sequence complementary to a target gene and a base sequence complementary to a probe used for forming a polymer. Note that, the assist probe as used herein refers to a probe having both a region capable of binding to a target substance to be detected and a sequence complementary to a probe used for forming a polymer, and serves to connect the target substance with a signal probe polymer. A method of using the assist probe has an advantage in that multiple genes may be simultaneously detected with the same set of multiple kinds of probes by preparing multiple assist probes whose region complementary to a gene to be tested are different from each other.
Patent Document 3 describes an assist probe having a sequence complementary to one region of a dimer probe, but does not mention any study on an assist probe capable of detecting a target substance with high sensitivity.
Patent Document 1: JP 2002-355081 A
Patent Document 2: WO 2003/029441
Patent Document 3: WO 2006/028162

### Disclosure of the Invention

### Problems to be solved by the Invention

In the light of actual circumstances of the above-mentioned related arts, the inventors of the present invention have made extensive studies to allow to enhance the detection sensitivity in the PALSAR method also corresponding to simultaneous detection of multiple kinds of genes. As a result, the inventors have found a method of designing an assist probe suitable for the PALSAR method.
It is an object of the present invention to provide a method of detecting a target substance using a novel assist probe, the method allowing to enhance the detection sensitivity and simultaneously detect multiple kinds of genes in the PALSAR method, a kit for detection used for the method, and a method of forming a signal probe polymer using the assist probe, and a signal probe polymer to be formed by the method.

### Means for solving the Problem

The inventors of the present invention have made extensive studies on designs of an assist probe in order to solve the above-mentioned problem, and as a result, have found a method of designing an assist probe optimal for the PALSAR method, and have completed the present invention.
That is, a method of detecting a target substance of the present invention is a method of detecting a target substance, including detecting a target substance by forming a signal probe polymer using one or more sets of paired dimer-forming probes or dimer probes formed of the paired dimer-forming probes, one or more kinds of crosslinking probes, and one or more kinds of assist probes, in which: each of the dimer-forming probes includes three regions, i.e., a 5'-side region, a central region, and a 3'-side region, central regions in each of the paired dimer-forming probes are complementary to each other, and the respective 3'-side regions and 5'-side regions in each of the paired dimer-forming probes are not complementary to each other unexceptionally; each of the crosslinking probes includes two regions, i.e., a 5'-side region and a 3'-side region; the 5'-side region in each of the dimer-forming probes is complementary to the 5'-side region in any one of the crosslinking probes and the 3'-side region in each of the dimer-forming probes is complementary to the 3'-side region in any one of the crosslinking probes; and the assist probes include a region complementary to a 5'-side region in one of the paired dimer-forming probes, a region complementary to the 3'-side region in the other of the paired dimer-forming probes, and a target region capable of binding to the target substance.

Preferably, the assist probes include one or more of one or more kinds of binding regions including a first region complementary to the 5'-side region in one of the paired dimer-forming probes and a second region complementary to the 3'-side region in the other of the paired dimer-forming probes, and a 5'-side in the first region is adjacent to a 3'-side in the second region in the binding region.

Preferably, the dimer-forming probes include one set of paired dimer-forming probes and the crosslinking probes include one set of paired crosslinking probes; each 5'-side region in the paired dimer-forming probes is complementary to the 5'-side region in either one of the paired crosslinking probes and each 5'-side region in the paired crosslinking probes is complementary to the 5'-side region in either one of the paired dimer-forming probes; and each 3'-side region in the paired dimer-forming probes is complementary to the 3'-side region in either one of the paired crosslinking probes and each 3'-side region in the paired crosslinking probes is complementary to the 3'-side region in either one of the paired dimer-forming probes.

Further, suitably, the dimer-forming probes include multiple sets of paired dimer-forming probes and the crosslinking probes include multiple sets of paired crosslinking probes; each 5'-side region in the multiple sets of paired dimer-forming probes is complementary to the 5'-side region in any one of the multiple sets of paired crosslinking probes and each 5'-side region in the multiple sets of paired crosslinking probes is complementary to the 5'-side region in any one of the multiple sets of paired dimer-forming probes; and each 3'-side region in the multiple sets of paired dimer-forming probes is complementary to the 3'-side region in any one of the multiple sets of paired crosslinking probes and each 3'-side region in the multiple sets of paired crosslinking probes is complementary to the 3'-side region in any one of the multiple sets of paired dimer-forming probes.

A method of forming a signal probe polymer of the present invention is a method of forming a signal probe polymer by reacting a target substance, one or more sets of paired dimer-forming probes or dimer probes formed of the paired dimer-forming probes, one or more kinds of crosslinking probes, and one or more kinds of assist probes, in which: each of the dimer-forming probes includes three regions, i.e., a 5'-side region, a central region, and a 3'-side region, central regions in each of the paired dimer-forming probes are complementary to each other and the respective 3'-side regions and 5'-side regions in each of the paired dimer-forming probes are not complementary to each other unexceptionally; each of the crosslinking probes includes two regions, i.e., a 5'-side region and a 3'-side region; the 5'-side region in each of the dimer-forming probes is complementary to the 5'-side region in any one of the crosslinking probes and the 3'-side region in each of the dimer-forming probes is complementary to the 3'-side region in any one of the crosslinking probes; and the assist probes include a region complementary to the 5'-side region in one of the paired dimer-forming probes, a region complementary to the 3'-side region in the other of the paired dimer-forming probes, and a target region capable of binding to the target substance.

A signal probe polymer of the present invention is formed by the above-mentioned method of the present invention.

A kit for detection of a target substance of the present invention is a kit for detection of a target substance, which includes one or more sets of paired dimer-forming probes or dimer probes formed of the paired dimer-forming probes, one or more kinds of crosslinking probes, and one or more kinds of assist probes, in which: each of the dimer-forming probes includes three regions, i.e., a 5'-side region, a central region, and a 3'-side region, central regions in each of the paired dimer-forming probes are complementary to each other and the respective 3'-side regions and 5'-side regions in the paired dimer-forming probes are not complementary to each other unexceptionally; each of the crosslinking probes includes two regions, i.e., a 5'-side region and a 3'-side region; the 5'-side region in each of the dimer-forming probes is complementary to the 5'-side region in any one of the crosslinking probes and the 3'-side region in each of the dimer-forming probes is complementary to the 3'-side region in any one of the crosslinking probes; and the assist probes include a region complementary to the 5'-side region in one of the paired dimer-forming probes, a region complementary to the 3'-side region in the other of the paired dimer-forming probes, and a target region capable of binding to the target substance.

### Effects of the Invention

According to the present invention, it is possible to remarkably enhance the detection sensitivity in detecting a target substance using the PALSAR method. In the present invention, it is also possible to simultaneously detect multiple target substances by changing a target region in the assist probe.

### Brief Description of the Drawings

FIG. 1 is a schematic diagram illustrating one example of a kit for detection of the present invention. FIG. 1(a) illustrates a first example of a dimer probe, FIG. 1(b) illustrates a first example of a crosslinking probe, and FIG. 1(c) illustrates a first example of an assist probe.
FIG. 2 is a schematic diagram illustrating one example of binding aspects of a target substance, the assist probe, and the dimer probe in a method of detecting a target substance using the kit for detection described in FIG. 1.
FIG. 3 is a schematic diagram illustrating a first example of the method of detecting a target substance of the present invention.
FIG. 4 is a schematic diagram illustrating another example of the crosslinking probe to be used together with the dimer probe described in FIG. 1.
FIG. 5 is a schematic diagram illustrating a second example of the method of detecting a target substance of the present invention.
FIG. 6 is a schematic diagram illustrating a third example of the dimer probe and the crosslinking probe used for the present invention.
FIG. 7 is a schematic diagram illustrating a third example of the method of detecting a target substance of the present invention.
FIG. 8 is a schematic diagram illustrating a second example of the assist probe.
FIG. 9 is a schematic diagram illustrating a binding aspect of the dimer probe to the assist probe described in FIG. 8.
FIG. 10 is a schematic diagram illustrating a third example of the assist probe.
FIG. 11 is a schematic diagram illustrating a binding aspect the dimer probe to the assist probe described in FIG. 10.
FIG. 12 is a schematic diagram illustrating one example of two sets of dimer probes used for the present invention.
FIG. 13 is a schematic diagram illustrating one example of two sets of paired crosslinking probes to be used together with the dimer probes described in FIG. 12.

### Description of Symbols

10a, 10b: dimer probes, 11a to 11d: dimer-forming probes, 12a to 12d: crosslinking probes, 13: a hydrogen bond, 14a to 14c: assist probes, 16: a target substance, 18: a signal probe polymer, 20a, 20b: dimer probes, 21a to 21d: dimer-forming probes, 22a to 22d: crosslinking probes

### Best Mode for carrying out the Invention

Hereinafter, embodiments of the present invention are described with reference to the accompanying drawings, which are for illustrative purposes only, and it will be appreciated that various variations may be made without departing from the technical idea of the present invention.

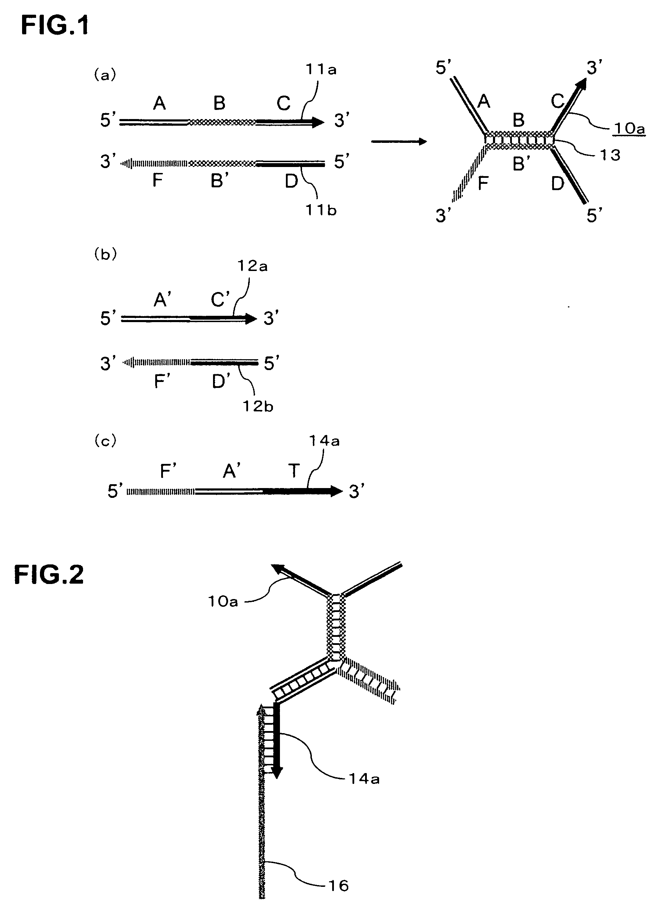

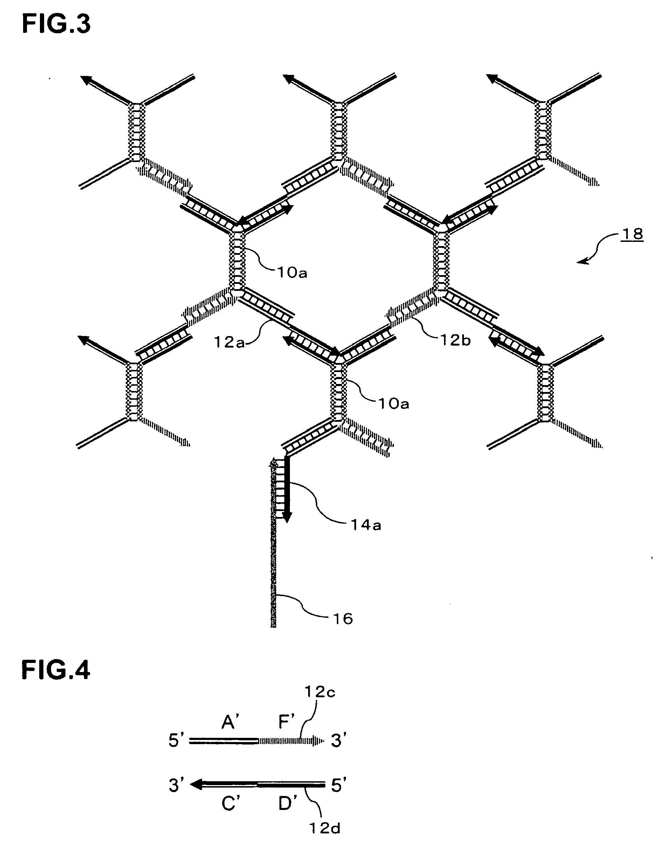

FIG. 1 is a schematic diagram illustrating a first example of a kit for detection of a target substance of the present invention. FIG. 2 is a schematic diagram illustrating one example of binding aspects of a target substance, an assist probe, and a dimer probe in a method of detecting a target substance using the kit for detection described in FIG. 1. FIG. 3 is a schematic diagram illustrating a first example of a method of detecting a target substance using the kit for detection described in FIG. 1.

The kit for detection of a target substance of the present invention includes one or more sets of paired dimer-forming probes or dimer probes formed of the paired dimer-forming probes, one or more kinds of crosslinking probes, and one or more kinds of assist probes.
FIG. 1 illustrates a first example in which one set of paired dimer-forming probes, one set of paired crosslinking probes, and one kind of assist probe are used.

FIG. 1(a) illustrates one set of paired dimer-forming probes (a first dimer-forming probe 11a and a second dimer-forming probe 11b) and a dimer probe 10a formed of the paired dimer-forming probes 11a, 11b.
As illustrated in FIG. 1(a), the paired dimer-forming probes are made up of two kinds of single strand nucleic acid probes (the first dimer-forming probe 11a and the second dimer-forming probe 11b), and each dimer-forming probe 11a, 11b includes at least three regions, i.e., a central region, a 5'-side region located at the 5'-side from the central region, and a 3'-side region located at the 3'-side from the central region. In FIG. 1, the 5'-side region, the central region, and the 3'-side region in the first dimer-forming probe 11a are represented by a region A, a region B, and a region C, respectively, and the 5'-side region, the central region, and the 3'-side region in the second dimer-forming probe 11b are represented by a region D, a region B', and a region F, respectively. The central regions (regions B and B') in the first dimer-forming probe 11a and the second dimer-forming probe 11b are complementary to each other. Four regions, i.e., the 5'-side regions (regions A and D) and the 3'-side regions (regions C and F) in the first dimer-forming probe 11a and in the second dimer-forming probe 11b are not complementary to each other unexceptionally. One set of dimer probe 10a is formed by hybridizing both dimer-forming probes. In the figure, a hydrogen bond is represented by 13.

In the present invention, a dimer probe formed by preliminarily hybridizing paired dimer-forming probes with each other may be used, or the dimer-forming probes may be used directly, but it is preferred to use a dimer probe.

When multiple sets of paired dimer-forming probes are used, each set of paired dimer-forming probes is constructed in the same way as in the above-mentioned one set. The multiple sets of paired dimer-forming probes form the same number of sets of dimer probes.

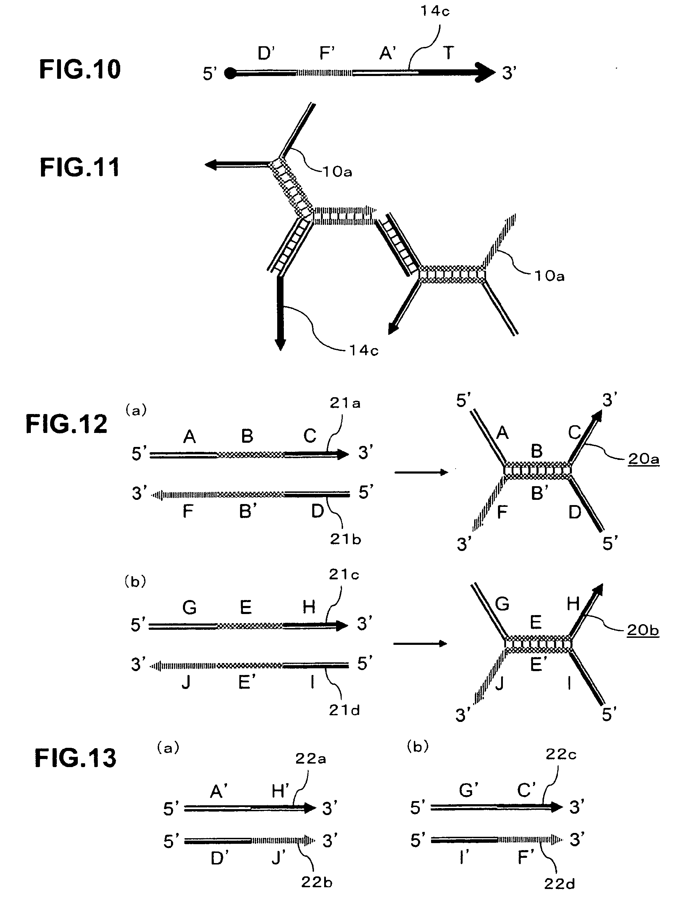

FIG. 12 is a schematic diagram illustrating one example of two sets of paired dimer-forming probes and two sets of dimer probes formed of the paired dimer-forming probes. FIG. 12(a) illustrates a first set of paired dimer-forming probes (a first dimer-forming probe 21a and a second dimer-forming probe 21b) and a dimer probe 20a formed of the paired dimer-forming probes 21a, 21b, and FIG. 12(b) illustrates a second set of paired dimer-forming probes (a first dimer-forming probe 21c and a second dimer-forming probe 21d) and a dimer probe 20b formed of the paired dimer-forming probes 21c, 21d.
In FIG. 12, the 5'-side region, the central region, and the 3'-side region in the first dimer-forming probe 21a in the first set are represented by the region A, the region B, and the region C, respectively, and the 5'-side region, the central region, and the 3'-side region in the second dimer-forming probe 21b in the first set are represented by the region D, the region B', and the region F, respectively. The 5'-side region, the central region, and the 3'-side region in the first dimer-forming probe 21c in the second set are represented by a region G, a region E, and a region H, respectively, and the 5'-side region, the central region, and the 3'-side region in the second dimer-forming probe 21d in the second set are represented by a region I, a region E' and a region J, respectively. The central regions (regions B and B' or regions E and E') in the first and second dimer-forming probes in each set are complementary to each other. Four regions, i.e., the 5'-side regions and the 3'-side regions (regions A, C, D, and F, regions G, H, I, and J) in the first and second dimer-forming probes in each set are not complementary to each other unexceptionally. Two sets of dimer probes 20a, 20b are formed by hybridizing paired dimer-forming probes 21a, 21b, 21c, 21d in respective sets. It is preferred that the 3'-side regions and the 5'-side regions in the dimer-forming probes to be used be not complementary to each other unexceptionally.

In the present invention, the crosslinking probe is one or more kinds of single strand nucleic acid probes which can crosslink the dimer probes formed of the dimer-forming probes, and includes at least two regions. In the two regions, the region located at the 5'-side is referred to as a 5'-side region and the region located at the 3'-side is referred to as a 3'-side region. The 5'-side region in each dimer-forming probe is complementary to the 5'-side region in any one of the crosslinking probes, and the 3'-side region in each dimer-forming probe is complementary to the 3'-side region in any one of the crosslinking probes. By making such structure, the crosslinking probe can be bound to the dimer-forming probe so as to crosslink one or more kinds of multiple dimer probes formed of the dimer-forming probes, to thereby form an assembly substance of the probes (probe polymer).

When one set of paired dimer-forming probes is used, it is preferred to use one set of paired crosslinking probes. One set of paired dimer-forming probes (the first dimer-forming probe and the second dimer-forming probe) and one set of paired crosslinking probes (a first crosslinking probe and a second crosslinking probe) are constructed so that each 5'-side region in the paired dimer-forming probes is complementary to the 5'-side region in either one of the paired crosslinking probes and each 5'-side region in the paired crosslinking probes is complementary to the 5'-side region in either one of the paired dimer-forming probes, as well as each 3'-side region in the paired dimer-forming probes is complementary to the 3'-side region in either one of the paired crosslinking probes and each 3'-side region in the paired crosslinking probes is complementary to the 3'-side region in either one of the paired dimer-forming probes.

FIG. 1(b) is a schematic diagram illustrating one example of one set of paired crosslinking probes (a first crosslinking probe 12a and a second crosslinking probe 12b) to be used together with paired dimer-forming probes 11a, 11b described in FIG. 1(a).
For the crosslinking probe to be used together with one set of paired dimer-forming probes 11a, 11b described in FIG. 1(a), for example, as illustrated in FIG. 1(b), paired crosslinking probes in which the 5'-side region in the first crosslinking probe 12a is complementary to the 5'-side region (region A) in the first dimer-forming probe 11a, the 3'-side region in the first crosslinking probe 12a is complementary to the 3'-side region (region C) in the first dimer-forming probe 11a, the 5'-side region in the second crosslinking probe 12b is complementary to the 5'-side region (region D) in the second dimer-forming probe 11b, and the 3'-side region in the second crosslinking probe 12b is complementary to the 3'-side region (region F) in the second dimer-forming probe 11b are suitable. A signal probe polymer 18 may be formed (FIG. 3) by hybridizing the dimer-forming probes 11a, 11b described in FIG. 1(a) with the crosslinking probes 12a, 12b described in FIG. 1(b).
Note that, as used herein, the region A' means a region having a base sequence complementary to the region A, the region B' means a region having a base sequence complementary to the region B, the region C' means a region having a base sequence complementary to the region C, the region D' means a region having a base sequence complementary to the region D, and the region F' means a region having a base sequence complementary to the region F.

FIG. 1 illustrates an example in which the 5'-side region and the 3'-side region in the first dimer-forming probe 11a are complementary to the 5'-side region and the 3'-side region in the first crosslinking probe 12a, respectively, and the 5'-side region and the 3'-side region in the second dimer-forming probe 11b are complementary to the 5'-side region and the 3'-side region in the second crosslinking probe 12b, respectively. However, the present invention encompasses any example in which the 5'-side region in one dimer-forming probe is complementary to the 5'-side region in one crosslinking probe and the 3'-side region in one dimer-forming probe is complementary to the 3'-side region in one crosslinking probe.

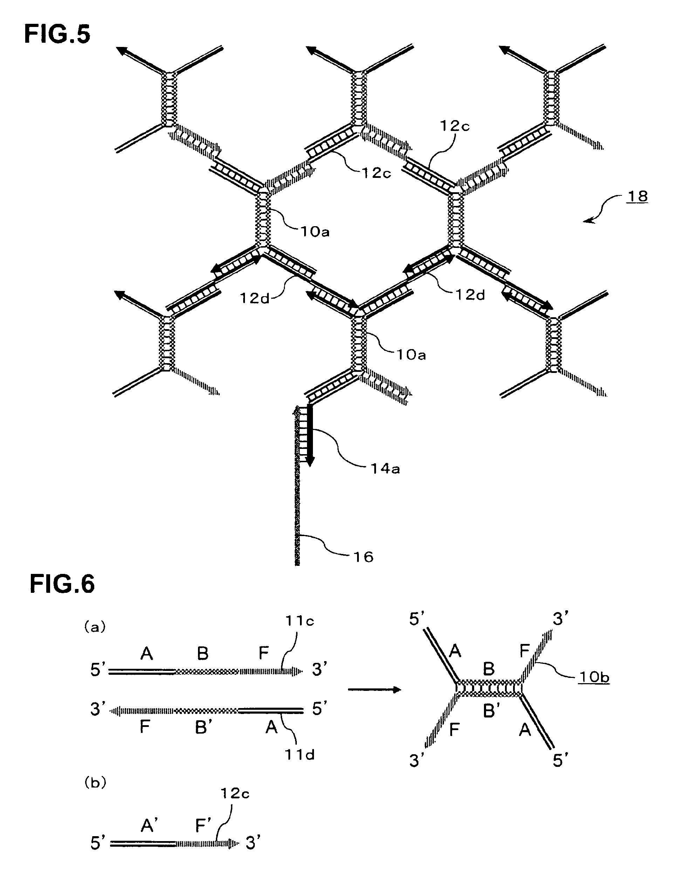

FIG. 4 is a schematic diagram illustrating another example of one set of paired crosslinking probes (a first crosslinking probe 12c and a second crosslinking probe 12d) to be used together with the paired dimer-forming probes 11a, 11b described in FIG. 1.
As illustrated in FIG. 4, another example of the paired crosslinking probes includes paired crosslinking probes in which the 5'-side region in the first crosslinking probe 12c is complementary to the 5'-side region (region A) in the first dimer-forming probe 11a, the 3'-side region in the first crosslinking probe 12c is complementary to the 3'-side region (region F) in the second dimer-forming probe 11b, the 5'-side region in the second crosslinking probe 12d is complementary to the 5'-side region (region D) in the second dimer-forming probe 11b, and the 3'-side region in the second crosslinking probe 12d is complementary to the 3'-side region (region C) in the first dimer-forming probe 11a. The signal probe polymer 18 may be formed (FIG. 5) by hybridizing the dimer-forming probes 11a, 11b described in FIG. 1(a) with the crosslinking probes 12c, 12d described in FIG. 4.

When multiple sets of paired dimer-forming probes are used, it is preferred to use the same number of sets of paired crosslinking probes. Specifically, it is suitable to use n sets (n represents an integer of 2 or more) of paired dimer-forming probes (i.e., 2n dimer-forming probes) and n sets of paired crosslinking probes (i.e., 2n crosslinking probes) and adopt such a structure that the 5'-side region of each of the dimer-forming probes is complementary to the 5'-side region in any one of the crosslinking probes and the 5'-side region in each of the crosslinking probes is complementary to the 5'-side region in any one of the dimer-forming probes, as well as the 3'-side region of each dimer-forming probe is complementary to the 3'-side region in any one of the crosslinking probes and the 3'-side region in each of the crosslinking probes is complementary to the 3'-side region in any one of the dimer-forming probes.

FIG. 13 is a schematic diagram illustrating one example of two sets of paired crosslinking probes 22a to 22d to be used together with two sets of paired dimer-forming probes 21a to 21d described in FIG. 12. FIG. 13(a) illustrates a first set of paired crosslinking probes (a first crosslinking probe 22a and a second crosslinking probe 22b), and FIG. 13(b) illustrates a second set of paired crosslinking probes (a first crosslinking probe 22c and a second crosslinking probe 22d).
As illustrated in FIG. 13, as the crosslinking probes to be used together with two sets of paired dimer-forming probes 21a to 21d described in FIG. 12, the two sets of paired crosslinking probes (i.e., four kinds of crosslinking probes) may be used and preferably constituted such that the 5'-side region of each dimer-forming probe is complementary to the 5'-side region in any one of the crosslinking probes and the 5'-side region in each crosslinking probe is complementary to the 5'-side region in any one of the dimer-forming probes, as well as the 3'-side region of each dimer-forming probe is complementary to the 3'-side region in any one of the crosslinking probe and the 3'-side region in each crosslinking probe is complementary to the 3'-side region in any one of the dimer-forming probes.

Specifically, as illustrated in FIG. 13, two sets of paired crosslinking probes are suitable in which the 5'-side region in the first crosslinking probe 22a in the first set is complementary to the 5'-side region (region A) in the first dimer-forming probe 21a in the first set, the 5'-side region in the second crosslinking probe 22b in the first set is complementary to the 5'-side region (region D) in the second dimer-forming probe 21b in the first set, the 5'-side region in the first crosslinking probe 22c in the second set is complementary to the 5'-side region (region G) in the first dimer-forming probe 21c in the second set, the 5'-side region in the second crosslinking probe 22d in the second set is complementary to the 5'-side region (region D) in the second dimer-forming probe 21d in the second set, the 3'-side region in the first crosslinking probe 22a in the first set is complementary to the 3'-side region in any one of four kinds of dimer-forming probes 21a to 21d (FIG. 13 illustrates a case of being complementary to the region H), the 3'-side region in the second crosslinking probe 22b in the first set is complementary to the 3'-side region in any one of four kinds of dimer-forming probes 21a to 21d excluding one selected for the crosslinking probe 22a in the first set (FIG. 13 illustrates a case of being complementary to the region J), the 3'-side region in the first crosslinking probe 22c in the second set is complementary to the 3'-side region in any one of four kinds of dimer-forming probes 21a to 21d excluding ones selected for the first and second crosslinking probes 22a, 22b in the first set (FIG. 13 illustrates a case of being complementary to the region C), and the 3'-side region in the second crosslinking probe 22d in the second set is complementary to the 3'-side regions in one of the four kinds of dimer-forming probes 21a to 21d excluding ones selected for the first and second crosslinking probes 22a, 22b in the first set and the first crosslinking probe 22c in the second set (FIG. 13 illustrates a case of being complementary to the region F). A signal probe polymer 18 can be formed by hybridizing the dimer-forming probes 21a to 21d described in FIG. 12 with the crosslinking probes 22a to 22d described in FIG. 13.
Note that, as used herein, the region E' means a region having a base sequence complementary to the region E, the region G' means a region having a base sequence complementary to the region G, the region H' means a region having a base sequence complementary to the region H, the region I' means a region having a base sequence complementary to the region I, and the region J' means a region having a base sequence complementary to the region J.

Note that FIG. 13 illustrates a case in which the 3'-side region in the first crosslinking probe 22a in the first set is complementary to the 3'-side region in the first dimer-forming probe 21c in the second set, the 3'-side region in the second crosslinking probe 22b in the first set is complementary to the 3'-side region in the second dimer-forming probe 21d in the second set, the 3'-side region in the first crosslinking probe 22c in the second set is complementary to the 3'-side region in the first dimer-forming probe 21a in the first set, and the 3'-side region in the second crosslinking probe 22d in the second set is complementary to the 3'-side region in the second dimer-forming probe 21b in the first set, but the combination of the 3'-side region in each crosslinking probe with the 3'-side region complementary to the region in the dimer-forming probe is not limited.

The length of each complementary region in the dimer-forming probe and the crosslinking probe is a length of at least 5 bases, preferably at least 8 bases, more preferably 10 to 100 bases, and still more preferably 12 to 30 bases. The lengths of the complementary regions in the respective probes are desirably the same as each other.

The base sequences of the respective regions in the dimer-forming probe and the crosslinking probe are not particularly limited as long as a predetermined region has a complementary base sequence and a predetermined region has a non-complementary base sequence, but it is preferred that the bases at both termini in each region be guanine or cytosine. By making the bases at both termini in each region guanine or cytosine, a reaction time can be shortened, and further, a stable probe polymer can be formed at lower reaction temperatures, to thereby enhance the workability and detection sensitivity.

In the present invention, the non-complementary base sequence may be any of base sequences which are not hybridized with each other, and also includes the identical base sequences.

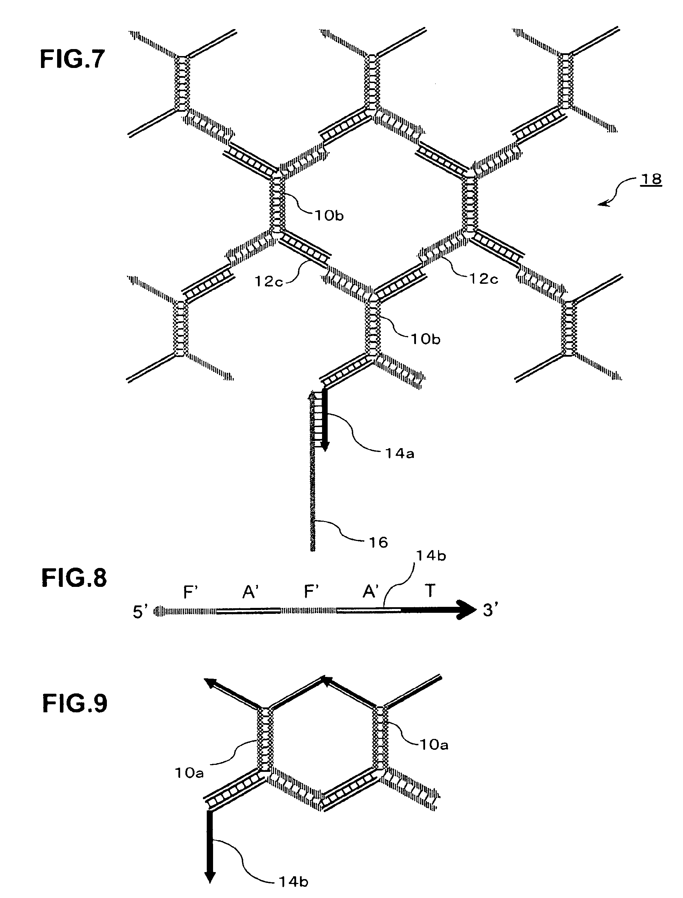

FIG. 6 is a schematic diagram illustrating a second example of one set of paired dimer-forming probes and one kind of paired crosslinking probes used for the present invention. FIG. 6(a) illustrates a dimer probe represented by 10b, and the example in which a dimer has been formed using two kinds of dimer-forming probes 11c, 11d, which have the same base sequences in their 3'-side regions and 5'-side regions. That is, in the dimer probe 10a in FIG. 1, the region A and the region D were allowed to have the same base sequence, and the region C and the region F were allowed to have the same base sequence. By making such structure, as illustrated in FIG. 6(b), the paired crosslinking probes to be used together with the dimer probe 10b are the same, and one kind of crosslinking probe 12c is used. The signal probe polymer 18 can be formed (FIG. 7) by hybridizing the dimer-forming probes 11c, 11d with the crosslinking probe 12c as described in FIG. 6.

The assist probe of the present invention has a region complementary to the 5'-side region in one dimer-forming probe in at least one set of paired dimer-forming probes, a region complementary to the 3'-side region in the other dimer-forming probe in the paired dimer-forming probes, and a target region capable of binding to a target substance, and is bound to two single strand regions at one end of at least one dimer probe.

FIG. 1(c) illustrates one example of the assist probe of the present invention to be used together with the one set of paired dimer-forming probes 11a, 11b described in FIG. 1(a). In FIG. 1(c), a first example of the assist probe of the present invention is represented by 14a, and the assist probe has a region (a region A') having a sequence complementary to the 5'-side region (a region A) in the first dimer-forming probe 11a of the dimer probe 10a, a region (a region F') having a sequence complementary to the 3'-side region (a region F) in the second dimer-forming probe 11b of the dimer probe 10a, and a target region (a region T) capable of binding to a target substance. In the assist probe 14a, the 3'-side of the region F' is adjacent to the 5'-side of the region A'.

In the assist probe of the present invention, relative positions of the respective regions are not particularly limited, but it is preferred that the respective regions binding to the dimer-forming probes be adjacent to each other as illustrated in FIG. 1. In the present invention, the region including a region (a first region) complementary to the 5'-side region in one probe in the set of paired dimer-forming probes and a region (a second region) complementary to the 3'-side region in the other probe in the paired dimer-forming probes is referred to as a binding region. In the binding region, it is more preferred that the 5'-side of the first region (e.g., a region A') complementary to the 5'-side region in one probe in paired dimer-forming probes be adjacent to the 3'-side of the second region (e.g., a region F') complementary to the 3'-side region in the other probe in the paired dimer-forming probes.

Specifically, when the dimer probe 10a described in FIG. 1 is used, it is preferred that the assist probe includes one or more cycles of one or more kinds of binding regions selected from the group consisting of a region F'A' (a region made up of the region F' and the region A' and in which the 3'-side of the region F' is adjacent to the 5'-side of the region A') and a region C'D' (a region made up of the region C' and the region D' and in which the 3'-side of the region C' is adjacent to the 5'-side of the region D'). By making such structure, as illustrated in FIG. 2, the assist probe can be bound consecutively to two single strand regions at one end in the dimer probe, which allows a target substance to be detected with high sensitivity.

The target region may be appropriately selected depending on a target substance in the assist probe of the present invention. When the target substance is a nucleic acid, it is preferred to adopt such a structure as the target region has a base sequence complementary to a target nucleic acid. When the target substance is a protein such as an antigen, it is preferred that a substance such as an antibody specifically binding to the target substance be bound directly or indirectly. Note that FIG. 2 illustrates an example in which the assist probe 14a is directly bound to a target substance 16, but the assist probe 14a may be bound indirectly to the target substance 16 via other nucleic acid probes and the like.
It is preferred that the target region (a region T) be located at the end portion in the assist probe. Further, the target region is suitably adjacent to a region binding to the dimer probe. Note that FIG. 1 illustrates an example in which the target region (a region T) is formed at the 3' end, but the target region (a region T) may be formed at the 5' end.

As illustrated in FIG. 3, the signal probe polymer 18 made up of a complex including a polymer formed of the dimer probe 10a and the crosslinking probes 12a and 12b as described in FIG. 1, the assist probe 14a, and the target substance 16 is formed by reacting the dimer probe 10a, the crosslinking probes 12a and 12b, the assist probe 14a, and the target substance 16.
In the present invention, the dimer probe, the crosslinking probe, the assist probe, and the target substance can be reacted simultaneously, but it is preferred that the target substance be reacted with the assist probe and subsequently, the dimer probe and the crosslinking probes be reacted with the reaction product. The probe polymer can also be formed by using the dimer-forming probe before forming the dimer probe and reacting the dimer-forming probe, the crosslinking probes, the assist probe, and the target substance.

The target substance 16 can be detected by detecting the formed signal probe polymer 18. The method of detecting a signal probe polymer is not particularly limited, but it is suitable to detect the signal probe polymer by using the dimer probe labeled with a labeling substance and detecting the labeling substance. The labeling substance includes, for example, a radioisotope, biotin, digoxigenin, a fluorescent substance, a luminescent substance, and a dyestuff as suitable examples. An intercalator such as ethidium bromide, OliGreen, and SYBR Green I may be bound to the formed signal probe polymer, and detected with fluorescence and the like.

FIG. 5 is a schematic diagram illustrating a second example of the method of detecting a target substance, and illustrates an example of the case where the crosslinking probes 12c and 12d described in FIG. 4 are used as the crosslinking probes in the kit for detection described in FIG. 1. FIG. 7 is a schematic diagram illustrating a third example of the method of detecting a target substance, and illustrates an example of the case where a kit for detection including the dimer probe 10b described in FIG. 6, the crosslinking probe 12c described in FIG. 6, and the assist probe 14a described in FIG. 1 are used.
In the kit for detection of the present invention, the sequence of the assist probe is determined depending on the constitution of the dimer probe and the target substance. As illustrated in FIGS. 1 to 7, in the kit for detection of the present invention, even if the dimer-forming probes and the crosslinking probe are changed, when the constitution of the 5'-side region in one dimer-forming probe in the dimer probe and the constitution of the 3'-side region in the other dimer-forming probe are the same, the same assist probe 14a can be used.

FIG. 8 is a schematic diagram illustrating a second example of the assist probe to be used together with the dimer probe described in FIG. 1, and FIG. 9 is a schematic diagram illustrating the binding of the dimer probe to the assist probe described in FIG. 8. FIG. 10 is a schematic diagram illustrating a third example of the assist probe to be used together with the dimer probe described in FIG. 1, and FIG. 11 is a schematic diagram illustrating the binding of the dimer probe to the assist probe described in FIG. 10.

The assist probe of the present invention includes one or more regions complementary to the single strand region in the formed dimer probes in addition to one region complementary to the 5'-side region in one dimer-forming probe in the dimer probe, one region complementary to the 3'-side region in the other dimer-forming probe in the dimer probe, and the target region capable of binding to the target substance, thereby being capable of binding one assist probe to two or more dimer probes.

Examples of the assist probe binding to two dimer probes are illustrated in FIGS. 8 to 11. In FIGS. 8 to 11, the assist probes 14b and 14c are constituted such that one or more additional regions (regions A', C', D', or F') complementary to the single strand regions (regions A, C, D, or F) of the dimer probe are further added to the assist probe 14a described in FIG. 1, i.e., are constituted so as to have one or more additional regions in addition to the region F'A' complementary to two regions at one end in the dimer probe 10a and capable of binding consecutively to the two regions and the target region T capable of binding to the target substance, and are constituted such that the target region T is located at the end region in the assist probe.

The number and kind of the additional regions in the assist probe are not particularly limited. For example, the additional region in the assist probe may be one region as illustrated in FIG. 10 and may be bound to one region in the dimer probe as illustrated in FIG. 11. However, it is preferred that the additional regions in the assist probe be two or more regions including one or more of two regions (e.g., a region F'A' and a region C'D') capable of binding to two regions at one end in the dimer probe as illustrated in FIG. 8, and be bound consecutively to the two regions at the one end in the dimer probe as illustrated in FIG. 9. The end region in the dimer probe to which the assist probe is bound is not limited, and two or more dimer probes may be bound at the same end side as illustrated in FIG. 9, or two or more dimer probes may be bound by combining different ends as illustrated in FIG. 11. When two or more sets of dimer probes are used, two or more sets of dimer probes may be bound to the assist probe.

It is preferred that the assist probe of the present invention alternately includes two regions (e.g., a region F' and a region A') complementary to two regions at one end in the dimer probe as illustrated in FIGS. 8 and 9. By making such a constitution, one assist probe can be bound alternately and consecutively to the two regions at the same end side in the same kind of the dimer probe, and can detect the target substance with extremely high sensitivity. In the sequence alternately including two regions complementary to two regions at one end in the dimer probe, an initial region and a last region can be selected from either the two regions, but it is preferred to constitute the assist probe so as to include multiple times of the regions (e.g., a region F'A' and a region C'D') capable of being consecutively bound to two regions at one end in the dimer probe as illustrated in FIGS. 8 and 9.

In the method of detecting a target substance of the present invention, it is preferred to use a carrier to which a substance capable of capturing a target substance has been immobilized. For example, when the target substance is a nucleic acid, it is preferred to use a nucleic acid probe having the sequence complementary to a site different from a binding site with the assist probe as a capture probe and use a carrier on the surface of which the capture probe has been immobilized. It is suitable to constitute the carrier such that the assist probe and the capture probe in an adjacent state are bound to the target nucleic acid.
As the carrier, it is preferred to use, for example, a fluorescent fine particle, a magnetic particle, a microplate, a microarray, a slide glass, and a substrate such as an electrically conductive substrate.

In the present invention, by combining multiple assist probes having different target regions T, it is possible to simultaneously detect multiple kinds of target substances. In the simultaneous detection of multiple kinds of target substances, the same dimer probes and the same crosslinking probes can be used, multiple kinds of assist probes and carriers capturing the target substance have only to be prepared, and thus the simultaneous detection is excellent in workability and low in cost.

### Examples

Hereinafter, the present invention is described more specifically by way of examples, but it goes without saying that those examples are for illustrative purposes only and should not be construed as restrictive ones.

### (Example 1)

One kind of dimer probe and two kinds of crosslinking probes described in FIG. 1 were used as the dimer probe and the crosslinking probe.

As nucleic acid probes used for forming the dimer probe, two kinds of oligonucleotide probes (a first dimer-forming probe-1 and a second dimer-forming probe-1) having the following base sequences, 5' ends of which had been labeled with digoxigenin, were used.
Base sequence of first dimer-forming probe-1 (5'-ABC-3')
5'-DIG-CATCTCTGCTGGTC CCTCGGCTGCGTCG GTTCGCCATAGACG-3' (SEQ ID NO: 1)
Base sequence of second dimer-forming probe-1 (5'-DB'F-3')
5'-DIG-GCACATTCACACCG CGACGCAGCCGAGG CCTGACCTCTATGC-3' (SEQ ID NO: 2)

The first dimer-forming probe-1 and the second dimer-forming probe-1 each were dissolved at a final concentration of 500 pmol/mL in a solution [4xSSC, 0.2% SDS, 1% blocking reagent (manufactured by Roche)]. Subsequently the solution was heated at 94°C for 1 minute and reacted at 60°C for 2 hours to prepare a dimer probe solution.

As the crosslinking probes, two kinds of oligonucleotide probes (a first crosslinking probe-1 and a second crosslinking probe-1) having the following base sequences, 5' ends of which had been labeled with digoxigenin, were used.
Base sequence of first crosslinking probe-1 (5'-A'C'-3')
5'-GACCAGCAGAGATG CGTCTATGGCGAAC-3' (SEQ ID NO: 3)
Base sequence of second crosslinking probe-1 (5'-D'F'-3')
5'-CGGTGTGAATGTGC GCATAGAGGTCAGG-3' (SEQ ID NO: 4)

The first crosslinking probe-1 and the second crosslinking probe-1 each were dissolved at a final concentration of 500 pmol/mL in a solution [4xSSC, 0.2% SDS, 1% blocking reagent (manufactured by Roche)] to prepare a crosslinking probe solution.

A synthesized DNA (target oligo DNA) having the same base sequence as a rRNA from *Staphylococcus aureus* was used as a target substance.
Base sequence of target oligo DNA

A nucleic acid probe (an assist probe-1, an assist probe described in FIG. 1) having a region complementary to a 3'-side region of the second dimer-forming probe, a region complementary to a 5'-side region of the second dimer-forming probe, and a region complementary to the target oligo DNA sequentially from the 5'-side was used as the assist probe.
Base sequence of assist probe-1 (5'-F'A'-T₁-3') The assist probe-1 was dissolved at a final concentration of 25 pmol/mL in a solution [4×SSC, 0.2% SDS, 1% blocking reagent (manufactured by Roche), 20% formaldehyde, salmon sperm DNA (10 µg/mL)] to prepare an assist probe solution.

A nucleic acid probe having the sequence complementary to the target oligo DNA was used as a capture probe.
Base sequence of a capture probe The capture probe was immobilized in each of the wells in a strip well type 96-well microplate, which was then used for an experiment.

Subsequently, 50 µL of the target oligo DNA at each concentration (0.1, 1, or 10 fmol/mL) and 50 µL of the assist probe solution were added to the strip well type 96-well microplate prepared above, the microplate was tightly sealed with a plate sealer, and the contents in the microplate were reacted under a condition, at 20°C in the upper part and 55°C in the lower part of the microplate, for 45 minutes. After the reaction, the microplate was washed with a washing solution [50 mM Tris, 0.3 M NaCl, 0.01% Triton X-100, pH 7.6].

After washing, the washing solution was drained well, and 25 µL of a dimer probe solution, 25 µL of a crosslinking solution, and 50 µL of a solution [4×SSC, 0.2% SDS, 1% blocking reagent (manufactured by Roche)] were added to the 96-well microplate, which was then tightly sealed with a plate sealer. The contents in the microplate were reacted under the condition, at 20°C in the upper part and 55°C in the lower part of the microplate, for 30 minutes, and the microplate was then washed with a washing solution.

After washing the microplate, 100 µL of 15 mU/mL of ALP-labeled anti-digoxigenin (100 mM Tris, pH 7.5) were added to the microplate, followed by reaction in an incubator at 37°C. After washing the microplate, 100 µL of a luminescent substrate solution (CDP-Star manufactured by TROPIX, INC.) were added to the microplate, and after reaction in a dark place for 20 minutes, a luminescence intensity (RLU) was measured using a luminometer (Centro LB960 manufactured by Berthold). Results are shown in Table 1.

### (Example 2)

An experiment was carried out in the same way as in Example 1, except that an assist probe-2 having the following base sequence was used as the assist probe in place of the assist probe-1. The results are shown in Table 1.
Base sequence of assist probe-2 (5'-F'A'-F'A'-T₁-3')

### (Example 3)

An experiment was carried out in the same way as in Example 1, except that an assist probe-3 having the following base sequence was used as the assist probe in place of the assist probe-1. The results are shown in Table 1.
Base sequence of assist probe-3 (5'-A'-C'D'-T₁-3')

### (Experimental Example 1)

An experiment was carried out in the same way as in Example 1, except that a nucleic acid probe (an assist probe-4) having the region complementary to the 5'-side region in the first dimer-forming probe-1, and the region complementary to the target oligo DNA was used as the assist probe in place of the assist probe-1. The results are shown in Table 1.
Base sequence of assist probe-4 (5'-A'-T₁-3')

### (Experimental Example 2)

An experiment was carried out in the same way as in Example 1, except that a nucleic acid probe (an assist probe-5) having unexceptionally the same base sequence as the first dimer-forming probe-1 (i.e., made up of the region complementary to the 5'-side region in the first crosslinking probe-1, the region which was not bound to the crosslinking probe, and the region complementary to the 3'-side region in the first crosslinking probe-1) and the region complementary to the target oligo DNA was used as the assist probe in place of the assist probe-1. The results are shown in Table 1.
Base sequence of assist probe-5 (5'-ABC-T₁-3')

### (Experimental Example 3)

An experiment was carried out in the same way as in Example 1, except that a nucleic acid probe (an assist probe-8) having the region complementary to the 5'-side region in the first dimer-forming probe-1, the region which was not bound to the dimer probe and the crosslinking probe, the region complementary to the 3'-side region in the first dimer-forming probe-1, and the region complementary to the target oligo DNA was used as the assist probe in place of the assist probe-1. The results are shown in Table 1.
Base sequence of assist probe-8 (5'-A'EC'-T₁-3')

### (Experimental Example 4)

An experiment was carried out using a dimer probe having the sequence complementary to the target oligo DNA.
A dimer probe solution was prepared in the same way as in Example 1, except that a first dimer-forming probe-2 having the following base sequence, 5' end of which had been labeled with digoxigenin, was used in place of the first dimer-forming probe-1.
Base sequence of first dimer-forming probe-2 (5'-AB-T₂-3') A crosslinking probe solution was prepared in the same way as in Example 1, except that a first crosslinking probe-2 having the following base sequence, 5' end of which had been labeled with digoxigenin, was used in place of the first crosslinking probe-1.
Base sequence of the first crosslinking probe-2 (5'-A'-T₂'-3')
5'-DIG-GACCAGCAGAGATG GTCTTGCTGTCACT-3' (SEQ ID NO: 14)

An experiment was carried out in the same way as in Example 1, except that a solution [4×SSC, 0.2% SDS, 1% blocking reagent (manufactured by Roche), 20% formaldehyde, salmon sperm DNA (10 µg/mL)] containing no assist probe was used in place of the assist probe solution and further, the dimer probe solution and the crosslinking probe solution were changed as described above. The results are shown in Table 1.

### (Experimental Example 5)

An experiment was carried out in the same way as in Example 1, except that a solution [4xSSC, 0.2% SDS, 1% blocking reagent (manufactured by Roche), 20% formaldehyde, salmon sperm DNA (10 µg/mL)] containing 25 pmol/mL of the first dimer-forming probe-2 described in Experimental Example 4 was used in place of the assist probe solution and further, the dimer probe solution and the crosslinking probe solution were changed in the same way as in Experimental Example 4. The results are shown in Table 1.

**[Table 1]**

| Target oligo DNA concentration (fimol/mL) | Example 1 | Example 2 | Example 3 | Experimental Example 1 | Experimental Example 2 | Experimental Example 3 | Experimental Example 4 | Experimental Example 5 |
|---|---|---|---|---|---|---|---|---|
| 0.1 | 857 | 949 | 539 | 94 | 292 | 154 | 273 | 215 |
| 1 | 5596 | 9894 | 5581 | 1740 | 2445 | 1547 | 3648 | 3538 |
| 10 | 63,391 | 88,089 | 45,454 | 13,562 | 22,697 | 19,697 | 32,596 | 32,490 |

As shown in Table 1, the detection sensitivity in Examples 1 to 3 using the assist probe of the present invention was remarkably enhanced.

## Claims

1. A method of detecting a target nucleic acid, comprising detecting a target nucleic acid by forming a signal probe polymer using one or more sets of paired dimer-forming probes or dimer probes formed of the paired dimer-forming probes, one or more kinds of crosslinking probes, and one or more kinds of assist probes, wherein:
each of the dimer-forming probes comprises three regions, i.e., a 5'-side region, a central region, and a 3'-side region, central regions in each of the paired dimer-forming probes are complementary to each other, and the respective 3'-side regions and 5'-side regions in each of the paired dimer-forming probes are not complementary to each other;
each of the crosslinking probes comprises two regions, i.e., a 5'-side region and a 3'-side region;
the 5'-side region in each of the dimer-forming probes is complementary to the 5'-side region in any one of the crosslinking probes and the 3'-side region in each of the dimer-forming probes is complementary to the 3'-side region in any one of the crosslinking probes; and
the assist probes comprise one or more of one or more kinds of binding regions comprising a first region complementary to a 5'-side region in one of the paired dimer-forming probes, and a second region complementary to the 3'-side region in the other of the paired dimer-forming probes, wherein a 5'-side in the first region is adjacent to a 3'-side in the second region in the binding region, and a target region capable of binding to the target nucleic acid.

2. A method of detecting a target nucleic acid according to claim 1, wherein:
the dimer-forming probes comprise one set of paired dimer-forming probes and the crosslinking probes comprise one set of paired crosslinking probes;
each 5'-side region in the paired dimer-forming probes is complementary to the 5'-side region in either one of the paired crosslinking probes and each 5'-side region in the paired crosslinking probes is complementary to the 5'-side region in either one of the paired dimer-forming probes; and
each 3'-side region in the paired dimer-forming probes is complementary to the 3'-side region in either one of the paired crosslinking probes and each 3'-side region in the paired crosslinking probes is complementary to the 3'-side region in either one of the paired dimer-forming probes.

3. A method of detecting a target nucleic acid according to claim 1, wherein:
the dimer-forming probes comprise multiple sets of paired dimer-forming probes and the crosslinking probes comprise multiple sets of paired crosslinking probes;
each 5'-side region in the multiple sets of paired dimer-forming probes is complementary to the 5'-side region in any one of the multiple sets of paired crosslinking probes and each 5'-side region in the multiple sets of paired crosslinking probes is complementary to the 5'-side region in any one of the multiple sets of paired dimer-forming probes; and
each 3'-side region in the multiple sets of paired dimer-forming probes is complementary to the 3'-side region in any one of the multiple sets of paired crosslinking probes and each 3'-side region in the multiple sets of paired crosslinking probes is complementary to the 3'-side region in any one of the multiple sets of paired dimer-forming probes.

4. A method of forming a signal probe polymer by reacting a target nucleic acid, one or more sets of paired dimer-forming probes or dimer probes formed of the paired dimer-forming probes, one or more kinds of crosslinking probes, and one or more kinds of assist probes, wherein:
each of the dimer-forming probes comprises three regions, i.e., a 5'-side region, a central region, and a 3'-side region, central regions in each of the paired dimer-forming probes are complementary to each other and the respective 3'-side regions and 5'-side regions in each of the paired dimer-forming probes are not complementary to each other;
each of the crosslinking probes comprises two regions, i.e., a 5'-side region and a 3'-side region;
the 5'-side region in each of the dimer-forming probes is complementary to the 5'-side region in any one of the crosslinking probes and the 3'-side region in each of the dimer-forming probes is complementary to the 3'-side region in any one of the crosslinking probes; and the assist probes comprise one or more of one or more kinds of binding regions comprising a first region complementary to a 5'-side region in one of the paired dimer-forming probes, and a second region complementary to the 3'-side region in the other of the paired dimer-forming probes, wherein a 5'-side in the first region is adjacent to a 3'-side in the second region in the binding region, and a target region capable of binding to the target nucleic acid.

5. A signal probe polymer, which is formed by a method according to claim 4.

6. A kit for detection of a target nucleic acid, which comprises one or more sets of paired dimer-forming probes or dimer probes formed of the paired dimer-forming probes, one or more kinds of crosslinking probes, and one or more kinds of assist probes, wherein:
each of the dimer-forming probes comprises three regions, i.e., a 5'-side region, a central region, and a 3'-side region, central regions in each of the paired dimer-forming probes are complementary to each other and the respective 3'-side regions and 5'-side regions in the paired dimer-forming probes are not complementary to each other;
each of the crosslinking probes comprises two regions, i.e., a 5'-side region and a 3'-side region;
the 5'-side region in each of the dimer-forming probes is complementary to the 5'-side region in any one of the crosslinking probes and the 3'-side region in each of the dimer-forming probes is complementary to the 3'-side region in any one of the crosslinking probes; and the assist probes comprise one or more of one or more kinds of binding regions comprising a first region complementary to a 5'-side region in one of the paired dimer-forming probes, and a second region complementary to the 3'-side region in the other of the paired dimer-forming probes, wherein a 5'-side in the first region is adjacent to a 3'-side in the second region in the binding region, and a target region capable of binding to the target nucleic acid.

## Patentansprüche

1. Verfahren zum Detektieren einer Zielnukleinsäure, umfassend das Detektieren einer Zielnukleinsäure durch Bildung eines Signalsondenpolymers unter Verwendung eines oder mehrerer Sätze gepaarter Dimer-bildender Sonden oder aus den gepaarten Dimer-bildenden Sonden gebildete Dimer-Sonden, einer oder mehrerer Arten von Vernetzungssonden, und einer oder mehrerer Arten von Hilfssonden, wobei:
jede der Dimer-bildenden Sonden drei Regionen umfasst, d.h., eine Region auf der 5'-Seite, eine zentrale Region, und eine Region auf der 3'-Seite, wobei die zentralen Regionen in jeder der gepaarten Dimer-bildenden Sonden komplementär zueinander sind, und die jeweiligen Regionen auf der 3'-Seite und der 5'-Seite in jeder der gepaarten Dimer-bildenden Sonden nicht komplementär zueinander sind;
jede der Vernetzungssonden zwei Regionen umfasst, d.h., eine Region auf der 5'-Seite, und eine Region auf der 3'-Seite;
die Region auf der 5'-Seite in jeder der Dimer-bildenden Sonden komplementär ist zu der Region auf der 5'-Seite in allen Vernetzungssonden, und die Region auf der 3'-Seite in jeder der Dimer-bildenden Sonden komplementär ist zu der Region auf der 3'-Seite in allen Vernetzungssonden; und
die Hilfssonden eine oder mehrere von einer oder mehrerer Arten von Binderegionen umfassen, wobei die Binderegionen eine erste Region, welche komplementär ist zu der Region auf der 5'-Seite in einer der gepaarten Dimer-bildenden Sonden, und eine zweite Region, welche komplementär ist zu der Region auf der 3'-Seite in der anderen der gepaarten Dimer-bildenden Sonden, umfassen, wobei in der Binderegion die 5'-Seite in der ersten Region benachbart ist zu der 3'-Seite in der zweiten Region, und die Hilfssonden eine Zielregion umfassen, welche in der Lage ist, an die Zielnukleinsäure zu binden.

2. Verfahren zum Detektieren einer Zielnukleinsäure nach Anspruch 1, wobei:
die Dimer-bildenden Sonden einen Satz gepaarter Dimer-bildender Sonden umfassen und die Vernetzungssonden einen Satz gepaarter Vernetzungssonden umfassen;
jede Region auf der 5'-Seite in den gepaarten Dimer-bildenden Sonden komplementär ist zu der Region auf der 5'-Seite in den beiden gepaarten Vernetzungssonden, und jede Region auf der 5'-Seite in den gepaarten Vernetzungssonden komplementär ist zu der Region auf der 5'-Seite in den beiden gepaarten Dimer-bildenden Sonden; und
jede Region auf der 3'-Seite in den gepaarten Dimer-bildenden Sonden komplementär ist zu der Region auf der 3'-Seite in den beiden gepaarten Vernetzungssonden, und jede Region auf der 3'-Seite in den gepaarten Vernetzungssonden komplementär ist zu der Region auf der 3'-Seite in den beiden gepaarten Dimer-bildenden Sonden.

3. Verfahren zum Detektieren einer Zielnukleinsäure nach Anspruch 1, wobei:
die Dimer-bildenden Sonden multiple Sätze gepaarter Dimer-bildender Sonden umfassen, und die Vernetzungssonden multiple Sätze gepaarter Vernetzungssonden umfassen;
jede Region auf der 5'-Seite in den multiplen Sätzen gepaarter Dimer-bildender Sonden komplementär ist zu der Region auf der 5'-Seite in allen multiplen Sätzen gepaarter Vernetzungssonden, und jede Region auf der 5'-Seite in den multiplen Sätzen gepaarter Vernetzungssonden komplementär ist zu der Region auf der 5'-Seite in allen multiplen Sätzen gepaarter Dimer-bildender Sonden; und
jede Region auf der 3'-Seite in den multiplen Sätzen gepaarter Dimer-bildender Sonden komplementär ist zu der Region auf der 3'-Seite in allen multiplen Sätzen gepaarter Vernetzungssonden, und jede Region auf der 3'-Seite in den multiplen Sätzen gepaarter Vernetzungssonden komplementär ist zu der Region auf der 3'-Seite in allen multiplen Sätzen gepaarter Dimer-bildender Sonden.

4. Verfahren zum Bilden eines Signalsondenpolymers durch Reagieren einer Zielnukleinsäure, eines oder mehrerer Sätze gepaarter Dimer-bildender Sonden oder aus den gepaarten Dimer-bildenden Sonden gebildete Dimer-Sonden, einer oder mehrerer Arten von Vernetzungssonden, und einer oder mehrerer Arten von Hilfssonden, wobei:
jede der Dimer-bildenden Sonden drei Regionen umfasst, d.h., eine Region auf der 5'-Seite, eine zentrale Region, und eine Region auf der 3'-Seite, wobei die zentralen Regionen in jeder der gepaarten Dimer-bildenden Sonden komplementär zueinander sind, und die jeweiligen Regionen auf der 3'-Seite und 5'-Seite in jeder der gepaarten Dimer-bildenden Sonden nicht komplementär zueinander sind;
jede der Vernetzungssonden zwei Regionen umfasst, d.h., eine Region auf der 5'-Seite, und eine Region auf der 3'-Seite;
die Region auf der 5'-Seite in jeder der Dimer-bildenden Sonden komplementär ist zu der Region auf der 5'-Seite in allen Vernetzungssonden, und die Region auf der 3'-Seite in jeder der Dimer-bildenden Sonden komplementär ist zu der Region auf der 3'-Seite in allen Vernetzungssonden; und
die Hilfssonden eine oder mehrere von einer oder mehrerer Arten von Binderegionen umfassen, wobei die Binderegionen eine erste Region, welche komplementär ist zu der Region auf der 5'-Seite in einer der gepaarten Dimer-bildenden Sonden, und eine zweite Region, welche komplementär ist zu der Region auf der 3'-Seite in der anderen der gepaarten Dimer-bildenden Sonden, umfassen, wobei in der Binderegion die 5'-Seite in der ersten Region benachbart ist zu der 3'-Seite in der zweiten Region, und die Hilfssonden eine Zielregion umfassen, welche in der Lage ist, an die Zielnukleinsäure zu binden.

5. Signalsondenpolymer, welches durch ein Verfahren nach Anspruch 4 gebildet wird.

6. Kit zur Detektion einer Zielnukleinsäure, welches einen oder mehrere Sätze gepaarter Dimer-bildender Sonden oder aus den gepaarten Dimer-bildenden Sonden gebildete Dimer-Sonden, eine oder mehrere Arten von Vernetzungssonden, und eine oder mehrere Arten von Hilfssonden umfasst, wobei:
jede der Dimer-bildenden Sonden drei Regionen umfasst, d.h., eine Region auf der 5'-Seite, eine zentrale Region, und eine Region auf der 3'-Seite, wobei die zentralen Regionen in jeder der gepaarten Dimer-bildenden Sonden komplementär zueinander sind, und die jeweiligen Regionen auf der 3'-Seite und 5'-Seite in jeder der gepaarten Dimer-bildenden Sonden nicht komplementär zueinander sind;
jede der Vernetzungssonden zwei Regionen umfasst, d.h., eine Region auf der 5'-Seite, und eine Region auf der 3'-Seite;
die Region auf der 5'-Seite in jeder der Dimer-bildenden Sonden komplementär ist zu der Region auf der 5'-Seite in allen Vernetzungssonden, und die Region auf der 3'-Seite in jeder der Dimer-bildenden Sonden komplementär ist zu der Region auf der 3'-Seite in allen Vernetzungssonden; und
die Hilfssonden eine oder mehrere von einer oder mehrerer Arten von Binderegionen umfassen, wobei die Binderegionen eine erste Region, welche komplementär ist zu der Region auf der 5'-Seite in einer der gepaarten Dimer-bildenden Sonden, und eine zweite Region, welche komplementär ist zu der Region auf der 3'-Seite in der anderen der gepaarten Dimer-bildenden Sonden, umfassen, wobei in der Binderegion die 5'-Seite in der ersten Region benachbart ist zu der 3'-Seite in der zweiten Region, und die Hilfssonden eine Zielregion umfassen, welche in der Lage ist, an die Zielnukleinsäure zu binden.

## Revendications

1. Procédé de détection d'un acide nucléique cible, comprenant la détection d'un acide nucléique cible par formation d'un polymère servant de sonde signal en utilisant un ou plusieurs jeux de sondes formant des dimères appariées ou de sondes dimères formées à partir des sondes formant des dimères appariées, un ou plusieurs types de sondes de réticulation, et un ou plusieurs types de sondes d'assistance, dans lequel :
chacune des sondes formant des dimères comprend trois régions, à savoir une région du côté 5', une région centrale, et une région du côté 3', les régions centrales dans chacune des sondes formant des dimères appariées étant complémentaires les unes des autres, et les régions du côté 3' et les régions du côté 5' respectives dans chacune des sondes formant des dimères appariées n'étant pas complémentaires les unes des autres ;
chacune des sondes de réticulation comprend deux régions, à savoir une région du côté 5' et une région du côté 3' ;
la région du côté 5' dans chacune des sondes formant des dimères est complémentaire de la région du côté 5' dans n'importe laquelle des sondes de réticulation et la région du côté 3' dans chacune des sondes formant des dimères est complémentaire de la région du côté 3' dans n'importe laquelle des sondes de réticulation ; et
les sondes d'assistance comprennent un ou plusieurs types parmi un ou plusieurs types de régions de liaison comprenant une première région complémentaire d'une région du côté 5' dans l'une des sondes formant des dimères appariées, et une seconde région complémentaire de la région du côté 3' dans l'autre des sondes formant des dimères appariées, où un côté 5' dans la première région est adjacent à un côté 3' dans la seconde région dans la région de liaison, et une région cible apte à se lier à l'acide nucléique cible.

2. Procédé de détection d'un acide nucléique cible selon la revendication 1, dans lequel :
les sondes formant des dimères comprennent un jeu de sondes formant des dimères appariées et les sondes de réticulation comprennent un jeu de sondes de réticulation appariées ;
chaque région du côté 5' dans les sondes formant des dimères appariées est complémentaire de la région du côté 5' dans n'importe laquelle des sondes de réticulation appariées et chaque région du côté 5' dans les sondes de réticulation appariées est complémentaire de la région du côté 5' dans n'importe laquelle des sondes formant des dimères appariées ; et
chaque région du côté 3' dans les sondes formant des dimères appariées est complémentaire de la région du côté 3' dans n'importe laquelle des sondes de réticulation appariées et chaque région du côté 3' dans les sondes de réticulation appariées est complémentaire de la région du côté 3' dans n'importe laquelle des sondes formant des dimères appariées.

3. Procédé de détection d'un acide nucléique cible selon la revendication 1, dans lequel :
les sondes formant des dimères comprennent de multiples jeux de sondes formant des dimères appariées et les sondes de réticulation comprennent de multiples jeux de sondes de réticulation appariées ;
chaque région du côté 5' dans les multiples jeux de sondes formant des dimères appariées est complémentaire de la région du côté 5' dans n'importe lequel des multiples jeux de sondes de réticulation appariées et chaque région du côté 5' dans les multiples jeux de sondes de réticulation appariées est complémentaire de la région du côté 5' dans n'importe lequel des multiples jeux de sondes formant des dimères appariées ; et
chaque région du côté 3' dans les multiples jeux de sondes formant des dimères appariées est complémentaire de la région du côté 3' dans n'importe lequel des multiples jeux de sondes de réticulation appariées et chaque région du côté 3' dans les multiples jeux de sondes de réticulation appariées est complémentaire de la région du côté 3' dans n'importe lequel des multiples jeux de sondes formant des dimères appariées.

4. Procédé de formation d'un polymère servant de sonde signal, par mise en réaction d'un acide nucléique cible, d'un ou de plusieurs jeux de sondes formant des dimères appariées ou de sondes dimères formées à partir des sondes formant des dimères appariées, d'un ou plusieurs types de sondes de réticulation, et d'un ou plusieurs types de sondes d'assistance, dans lequel :
chacune des sondes formant des dimères comprend trois régions, à savoir une région du côté 5', une région centrale, et une région du côté 3', les régions centrales dans chacune des sondes formant des dimères appariées étant complémentaires les unes des autres, et les régions du côté 3' et les régions du côté 5' respectives dans chacune des sondes formant des dimères appariées n'étant pas complémentaires les unes des autres ;
chacune des sondes de réticulation comprend deux régions, à savoir une région du côté 5' et une région du côté 3' ;
la région du côté 5' dans chacune des sondes formant des dimères est complémentaire de la région du côté 5' dans n'importe laquelle des sondes de réticulation et la région du côté 3' dans chacune des sondes formant des dimères est complémentaire de la région du côté 3' dans n'importe laquelle des sondes de réticulation ; et
les sondes d'assistance comprennent un ou plusieurs types parmi un ou plusieurs types de régions de liaison comprenant une première région complémentaire d'une région du côté 5' dans l'une des sondes formant des dimères appariées, et une seconde région complémentaire de la région du côté 3' dans l'autre des sondes formant des dimères appariées, où un côté 5' dans la première région est adjacent à un côté 3' dans la seconde région dans la région de liaison, et une région cible apte à se lier à l'acide nucléique cible.

5. Polymère servant de sonde signal, qui est formé par un procédé selon la revendication 4.

6. Trousse de détection d'un acide nucléique cible, qui comprend un ou plusieurs jeux de sondes formant des dimères appariées ou de sondes dimères formées à partir des sondes formant des dimères appariées, un ou plusieurs types de sondes de réticulation, et un ou plusieurs types de sondes d'assistance, dans laquelle :
chacune des sondes formant des dimères comprend trois régions, à savoir une région du côté 5', une région centrale, et une région du côté 3', les régions centrales dans chacune des sondes formant des dimères appariées étant complémentaires les unes des autres, et les régions du côté 3' et les régions du côté 5' respectives dans les sondes formant des dimères appariées n'étant pas complémentaires les unes des autres ;
chacune des sondes de réticulation comprend deux régions, à savoir une région du côté 5' et une région du côté 3' ;
la région du côté 5' dans chacune des sondes formant des dimères est complémentaire de la région du côté 5' dans n'importe laquelle des sondes de réticulation et la région du côté 3' dans chacune des sondes formant des dimères est complémentaire de la région du côté 3' dans n'importe laquelle des sondes de réticulation ; et
les sondes d'assistance comprennent un ou plusieurs types parmi un ou plusieurs types de régions de liaison comprenant une première région complémentaire d'une région du côté 5' dans l'une des sondes formant des dimères appariées, et une seconde région complémentaire de la région du côté 3' dans l'autre des sondes formant des dimères appariées, où un côté 5' dans la première région est adjacent à un côté 3' dans la seconde région dans la région de liaison, et une région cible apte à se lier à l'acide nucléique cible.
